(19) Europäisches Patentamt
European Patent Office
Office européen des brevets

(11) **EP 1 428 792 A1**

(12) **EUROPEAN PATENT APPLICATION**

(43) Date of publication:
**16.06.2004 Bulletin 2004/25**

(51) Int Cl.[7]: **C01B 21/14**, C07C 249/08

(21) Application number: **02080204.7**

(22) Date of filing: **11.12.2002**

| (84) Designated Contracting States: | • **Benneker, Arno Herald** |
| **AT BE BG CH CY CZ DE DK EE ES FI FR GB GR** | **6439 AR Doenrade (NL)** |
| **IE IT LI LU MC NL PT SE SI SK TR** | |
| Designated Extension States: | (74) Representative: **Verhaegen, Ilse Maria M. et al** |
| **AL LT LV MK RO** | **DSM Intellectual Property** |
| | **Office Geleen** |
| (71) Applicant: **DSM IP Assets B.V.** | **P.O. Box 9** |
| **6411 TE Heerlen (NL)** | **6160 MA Geleen (NL)** |
| (72) Inventors: | |
| • **Oevering, Hendrik** | |
| **6181 BR Elsloo (NL)** | |

(54) **Process for mixing an acidic aqueous solution comprising hydroxyl ammonium and phosphate with nitric acid**

(57)     The invention relates to a process for mixing a first acidic aqueous solution comprising hydroxyl ammonium and phosphate with a second acidic aqueous solution comprising nitric acid at a temperature between 20 and 80 °C resulting in a third acidic aqueous solution comprising hydroxyl ammonium, phosphate and nitric acid, wherein in the third acidic aqueous solution the total acid concentration minus the phosphate concentration is lower than $0.523 * \ln([\text{hydroxyl ammonium}]/1.25) + 422/(T + 81)$ whereby [hydroxyl ammonium] is the concentration of hydroxyl ammonium in the third acidic aqueous solution, T is the temperature of the third acidic aqueous solution expressed in °C and all concentrations are expressed in mol/l.

Figure 1

**Description**

The invention relates to a process for mixing a first acidic aqueous solution comprising hydroxyl ammonium and phosphate with a second acidic aqueous solution comprising nitric acid at a temperature between 20 and 80 °C resulting in a third acidic aqueous solution comprising hydroxyl ammonium, phosphate and nitric acid. The invention further relates to a process for mixing the third acidic aqueous solution with an acidic aqueous solution comprising nitric acid at a temperature between 20 and 80 °C resulting in a fourth acidic aqueous solution comprising hydroxyl ammonium, phosphate and nitric acid.

Such a process is known from CN-A-1281849. In the process described in CN-A-1281849 a static mixer is used for mixing both solutions. However, in the mixed solution decomposition of hydroxyl ammonium may still occur. The disadvantage of decomposition of hydroxyl ammonium in an acidic aqueous solution comprising hydroxyl ammonium, phosphate and nitric acid is loss of hydroxyl ammonium. Hydroxyl ammonium may be produced by a catalytic reduction of nitrate with hydrogen, subsequently cyclohexanone oxime may be produced from hydroxyl ammonium and cyclohexanone. In such a process the aqueous reaction medium which is cycled between those production processes has to be enriched with nitrate ions before the production of hydroxyl ammonium. Enrichment with nitrate ions generally is performed by mixing the aqueous reaction medium with an acidic aqueous solution comprising nitric acid.

The object of the present invention is to provide a process for mixing a first acidic aqueous solution comprising hydroxyl ammonium and phosphate with a second acidic aqueous solution comprising nitric acid at a temperature between 20 and 80 °C resulting in a third acidic aqueous solution comprising hydroxyl ammonium, phosphate and nitric acid, in which decomposition of hydroxyl ammonium in the third acidic aqueous solution is prevented or at least reduced. Optionally this third acidic aqueous solution may be enriched further with nitric acid by mixing the third acidic aqueous solution with an acidic aqueous solution comprising nitric acid resulting in a fourth acidic aqueous solution. It is a further object of the present invention to prevent or at least reduce decomposition of hydroxyl ammonium in the fourth acidic aqueous solution.

This object is achieved in that in the third acidic aqueous solution the total acid concentration minus the phosphate concentration is lower,than $0.523 * \ln([\text{hydroxyl ammonium}]/1.25) + 422/(T + 81)$ whereby [hydroxyl ammonium] is the concentration of hydroxyl ammonium in the third acidic aqueous solution, T is the temperature of the third acidic aqueous solution expressed in °C and all concentrations are expressed in mol/l.

The total acid concentration in the third acidic aqueous solution is the sum of the concentration of $H_3PO_4$ and the concentration of $HNO_3$. Preferably this total acid concentration is higher than 0.1 mol/l and lower than 6 mol/l.

Generally, the phosphate concentration in the third acidic aqueous solution is higher than 2.0 mol/l. Preferably, the phosphate concentration is such that no crystallization occurs, which depends amongst other things on the temperature and the concentration of other components in the third acidic aqueous solution. Generally, the phosphate concentration in the third acidic aqueous solution is lower than 8 mol/l, preferably lower than 5 mol/l. As used herein, the phosphate concentration is defined as the overall concentration of all phosphates, irrespective of the form in which they are present, expressec in mol per litre of aqueous solution. Phosphates may be present as $PO_4^{3-}$, $HPO_4^{2-}$, $H_2PO_4^{-}$, $H_3PO_4$, salts of $PO_4^{3-}$, $HPO_4^{2-}$, $H_2PO_4^{-}$, and/or combinations thereof. Preferably, the third acidic aqueous solution comprising hydroxyl ammonium and phosphate is a phosphate buffered solution.

The upper limit for the total acid concentration minus the phosphate concentration in the third acidic aqueous solution is determined by the hydroxyl ammonium concentration ([hydroxyl ammonium]) and the temperature (T in °C) in the third acidic aqueous solution. In the process of the invention the total acid concentration minus the phosphate concentration should be lower than $0.523 * \ln([\text{hydroxyl ammonium}]/1.25) + 422/(T + 81)$ whereby all concentrations are expressed in mol/l. Preferably, the hydroxyl ammonium concentration will be adjusted in order to obtain a value for the total acid concentration minus the phosphate concentration lower than $0.523 * \ln([\text{hydroxyl ammonium}]/1.25) + 422/(T + 81)$. Surprisingly, it has been found that decomposition of hydroxyl ammonium in the third acidic aqueous solution may be reduced or even prevented by increasing the hydroxyl ammonium concentration in the third acidic aqueous solution.

There is no specific upper limit for the hydroxyl ammonium concentration ir the third acidic aqueous solution. Preferably, the hydroxyl ammonium concentration in the third acidic aqueous solution is lower than 2.5 mol/l.

The temperature of the third acidic aqueous solution is between 20 and 80 °C. Preferably, the temperature is between 25 and 60 °C. More preferably, the temperature is between 30 and 40 °C

Mixing of the first acidic aqueous solution with the second acidic aqueous solution may be performed by continuous merging the two acidic aqueous solutions supplied in a continuous flow and/or by using a mixer, a flow or line mixer, an agitated vessel or a bubble column. Preferably the mixing is performed with a turbine stirrer or a static mixer.

Any acidic aqueous solution comprising hydroxyl ammonium and phosphate can be used as first acidic aqueous solution. E. g. an acidic aqueous solution leaving a cyclohexanon oxime reactor can be used as first acidic aqueous solution.

Generally, the phosphate concentration in the first acidic aqueous solution is higher than 2.0 mol/l. Preferably, the phosphate concentration is such that no crystallization occurs, which depends amongst other things on the temperature and the concentration of other components in the aqueous solution. Generally, the phosphate concentration in the first acidic aqueous solution is lower than 8 mol/l, preferably lower than 5 mol/l. As used herein, the phosphate concentration is defined as the overall concentration of all phosphates, irrespective of the form in which they are present, expressed in mol per litre of aqueous solution. Phosphates may be present as $PO_4^{3-}$, $HPO_4^{2-}$, $H_2PO_4^{-}$, $H_3PO_4$, salts of $PO_4^{3-}$, $HPO_4^{2-}$, $H_2PO_4^{-}$, and/or combinations thereof. Preferably, the first acidic aqueous solution comprising hydroxyl ammonium and phosphate is a phosphate buffered solution .

In general the hydroxyl ammonium concentration in the first acidic aqueous solution is higher than 0.002 mol/l.

Any acidic aqueous solution comprising nitric acid can be used as the second acidic aqueous solution. The second acidic aqueous solution comprising nitric acid may be obtained by absorbing and oxidizing nitrogen oxides in an aqueous solution. It is also possible to use a concentrated nitric acid solution as the second acidic aqueous solution. Preferably such a concentrated nitric acid solution contains 30-75 % by weight of nitric acid. In a preferred embodiment, at least a part of the second acidic aqueous solution is obtained by absorbing and oxidizing nitrogen oxides in a part of the first acidic aqueous solution. Such part of the second acidic aqueous solution may be mixed with the other part of the second acidic aqueous solution, which may be a concentrated nitric acid solution.

The second acidic aqueous solution may also be obtained by mixing an aqueous solution in which nitrogen oxides are being absorbed and oxidized with a part of the first acidic aqueous solution. Nitrogen oxides may be obtained from an ammonia-oxidation. The oxidation of ammonia and nitrogen oxides in an aqueous solution to prepare nitric acid can be represented by the following equations:

$$4\ NH_3 + 5\ O_2 \rightarrow 4\ NO + 6\ H_2O$$

$$2\ NO + O_2 \rightarrow 2\ NO_2$$

$$4\ NO_2 + O_2 + 2\ H_2O \rightarrow 4\ HNO_3$$

$$3\ NO_2 + H_2O \rightarrow 2\ HNO_3 + NO$$

There is no specific upper limit for the nitric acid concentration in the second acidic aqueous solution.

After mixing the first acidic aqueous solution with the second acidic aqueous solution resulting in the third acidic aqueous solution, the concentration of nitric acid in the third acidic aqueous solution may be further increased by mixing the third acidic aqueous solution with an acidic aqueous solution comprising nitric acid at a temperature between 20 and 80 °C resulting in a fourth acidic aqueous solution comprising hydroxyl ammonium, phosphate and nitric acid whereby the total acid concentration minus the phosphate concentration in the fourth acidic aqueous solution is lower than 0.523 ∗ ln([hydroxyl ammonium]/1.25) + 422/(T + 81) wherein [hydroxyl ammonium] is the concentration of hydroxyl ammonium in the fourth acidic aqueous solution, T is the temperature of the fourth acidic aqueous solution expressed in °C and all concentrations are expressed in mol/l.

The total acid concentration in the fourth acidic aqueous solution is the sum of the concentration of $H_3PO_4$ and the concentration of $HNO_3$. This total acid concentration can be measured by means of titration of the acidic aqueous solutions to a pH value of 4.2. Preferably this total acid concentration is higher than 0.1 mol/l and lower than 6 mol/l.

Generally, the phosphate concentration in the fourth acidic aqueous solution is higher than 2.0 mol/l. Preferably, the phosphate concentration is such that no crystallization occurs, which depends amongst other things on the temperature and the concentration of other components in the aqueous solution. Generally, the phosphate concentration in the fourth acidic aqueous solution is lower than 8 mol/l, preferably lower than 5 mol/l. As used herein, the phosphate concentration is defined as the overall concentration of all phosphates, irrespective of the form in which they are present, expressed in mol per litre of aqueous solution. Phosphates may be present as $PO_4^{3-}$, $HPO_4^{2-}$, $H_2PO_4^{-}$, $H_3PO_4$, salts of $PO_4^{3-}$, $HPO_4^{2-}$, $H_2PO_4^{-}$, and/or combinations thereof. Preferably, the fourth acidic aqueous solution comprising hydroxyl ammonium and phosphate is a phosphate buffered solution.

The upper limit for the total acid concentration minus the phosphate concentration in the fourth acidic aqueous solution is determined by the hydroxyl ammonium concentration ([hydroxyl ammonium]) and the temperature (T) in the fourth acidic aqueous solution. The total acid concentration minus the phosphate concentration is such that it is always lower than 0.523 ∗ ln([hydroxyl ammonium]/1.25) + 422/(T + 81) whereby all concentrations are expressed in mol/l.

There is no specific upper limit for the hydroxyl ammonium concentration in the fourth acidic aqueous solution. Preferably, the hydroxyl ammonium concentration in the fourth acidic aqueous solution is lower than 2.5 mol/l.

The temperature of the fourth acidic aqueous solution is between 20 and 80 °C. Preferably, the temperature is between 25 and 60 °C. More preferably, the temperature is between 30 and 40 °C

Mixing of the third acidic aqueous solution with an acidic aqueous solution comprising nitric acid may be performed by continuous merging the two acidic aqueous solutions supplied in a continuous flow and/or by using a mixer, a flow or line mixer, an agitated vessel or a bubble column. Preferably the mixing is performed with a turbine stirrer or a static mixer.

The third acidic aqueous solution obtained by mixing the first acidic aqueous solution with the second acidic aqueous solution comprising nitric acid and/or the fourth acidic aqueous solution obtained by mixing the third acidic aqueous solution with an acidic aqueous solution comprising nitric acid is preferably used as aqueous reaction medium for the production of hydroxyl ammonium. For that purpose the third or fourth acidic aqueous solution may be fed to a hydroxyl ammonium reactor in which hydroxyl ammonium is prepared by catalytic reduction of nitrate with hydrogen, according to the following equation:

$$2\,H_3PO_4 + NO_3^- + 3\,H_2 \rightarrow NH_3OH^+ + 2\,H_2PO4^- + 2\,H_2O$$

In an embodiment of the process according to the present invention the third acidic aqueous solution used as aqueous reaction medium for the production of hydroxyl ammonium, the aqueous reaction medium leaving the hydroxyl ammonium reactor is cycled to a cyclohexanone oxime reactor and back to the hydroxyl ammonium reactor. In the cyclohexanone oxime reactor cyclohexanone oxime is produced from hydroxyl ammonium and cyclohexanone according to the following equation:

$$NH_3OH^+ + H_2PO_4^- + C_6H_{10}O \rightarrow C_6H_{10}NOH + H_3PO_4 + H_2O$$

Generally, the aqueous reaction medium leaving the cyclohexanone oxime reactor may comprise unreacted hydroxyl ammonium. However, to be used again as aqueous reaction medium for the production of hydroxyl ammonium the amount of nitrate ions has to be increased. Generally, the amount of nitrate ions is increased by mixing the aqueous reaction medium cycled back from the cyclohexanone oxime reactor to the hydroxyl ammonium reactor with an acidic aqueous nitric acid solution. In one embodiment the aqueous reaction medium cycled back from the cyclohexanone oxime reactor to the hydroxyl ammonium reactor is used as the first acidic aqueous solution and the acidic aqueous nitric acid solution is used as the second acidic aqueous solution. In another embodiment the mixing of the first and second acidic aqueous solution resulting in the third acidic aqueous solution is performed before the third acidic aqueous solution is fed to the hydroxyl ammonium reactor. In still another embodiment this third acidic aqueous solution is mixed with an acidic aqueous solution comprising nitric acid resulting in the fourth acidic aqueous solution before this fourth acidic aqueous solution is fed to the hydroxyl ammonium reactor.

In preferred embodiment the mixing of the first and second acidic aqueous solution or the mixing of the third acidic aqueous solution with an acidic aqueous solution comprising nitric acid is performed in a static mixer.

In another embodiment the mixing of the first acidic aqueous solution with the second acidic aqueous solution comprising nitric acid is performed in the hydroxyl ammonium reactor. In this embodiment the third acidic aqueous solution may be mixed with an acidic aqueous solution comprising nitric acid which is supplied to the hydroxyl ammonium reactor, resulting in the fourth acidic aqueous solution. The mixing in the hydroxyl ammonium reactor preferably is performed by using a bubble column.

Preferably, the concentration of hydroxyl ammonium in the third or fourth acidic aqueous solution is increased by adding hydroxyl ammonium to the first acidic aqueous solution before it is mixed with the second acidic aqueous solution comprising nitric acid. In an embodiment in which the third acidic aqueous solution is mixed with an acidic aqueous solution comprising nitric acid, an additional amount of hydroxyl ammonium may also be fed to the third acidic aqueous solution. In the embodiment in which the third or fourth acidic aqueous solution is mixed with an acidic aqueous solution comprising nitric acid in the hydroxyl ammonium reactor the first or third acidic aqueous solution is preferably enriched with hydroxyl ammonium in the hydroxyl ammonium reactor.

In a preferred embodiment in which a part of the first acidic aqueous solution is tapped for absorbing and oxidizing nitrogen oxides hydroxyl ammonium is added to the first acidic aqueous solution before it is mixed with the second acidic aqueous solution but after a part is tapped for absorbing and oxidizing nitrogen oxides. In the acidic aqueous solution in which nitrogen oxides are being absorbed and oxidized hydroxyl ammonium may decompose. In a more preferred embodiment a part of the aqueous reaction medium leaving the hydroxyl ammonium reactor is used to add hydroxyl ammonium to the first acidic aqueous solution. In still another embodiment a part of the aqueous reaction medium leaving the hydroxyl ammonium reactor is used to add hydroxyl ammonium to the third acidic aqueous solution. In such embodiments any part of the aqueous reaction medium leaving the hydroxyl ammonium reactor may be used to add hydroxyl ammonium to the first or third acidic aqueous solution. Preferably, 1-50 % by volume, more preferably, 5-30 % by volume may be used.

Brief Description of the Drawing

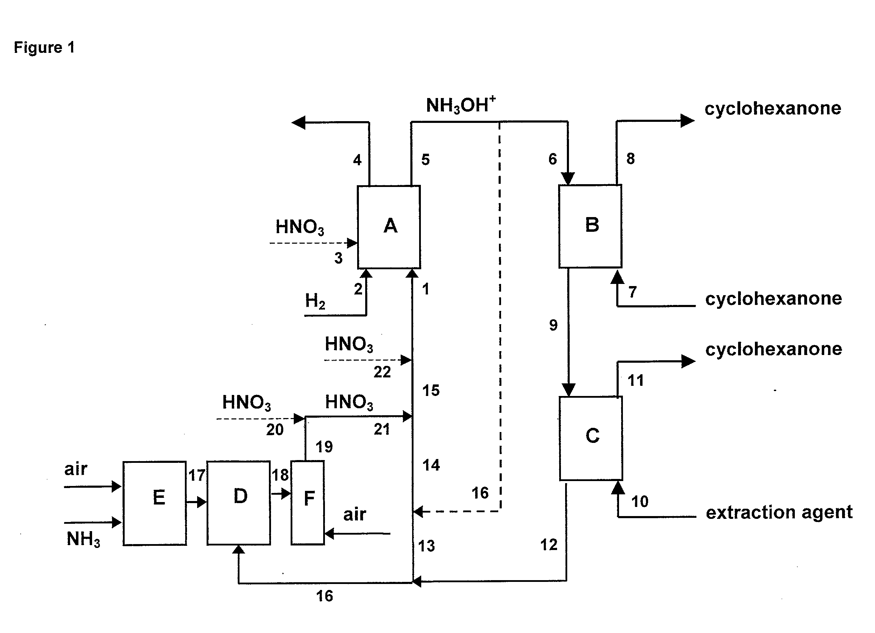

FIG. 1 is a schematic diagram of an embodiment of the process according to the present invention.

Description of an embodiment

Referring to FIG. 1, A represents the hydroxyl ammonium reactor. B represents the cyclohexanone oxime reactor. To reactor A, containing catalyst, an acidic aqueous solution comprising hydroxyl ammonium, phosphate and nitric acid is fed as aqueous reaction medium through line 1 and hydrogen is fed through line 2; optionally an additional acidic aqueous solution comprising nitric acid is fed to reactor A through line 3; unreacted hydrogen is discharged, with any other gases, via line 4. In the hydroxyl ammonium reactor A, the aqueous reaction medium is enriched in hydroxyl ammonium. This aqueous reaction medium being enriched in hydroxyl ammonium leaves reactor A via line 5 and is cycled to the cyclohexanone oxime reactor B via line 6. The cyclohexanone to be converted is fed in an organic solvent to reactor B via line 7. The largest part of cyclohexanone oxime produced and dissolved in the organic solvent is removed from the system via line 8. The aqueous reaction medium leaving the cyclohexanone oxime reactor through line 9 is extracted in an extraction zone C. An extraction agent, an organic solvent, enters extraction zone C through line 10. Within extraction zone C, additional cyclohexanone oxime is removed from the aqueous reaction medium and carried out of zone C in the organic solvent through line 11.

The aqueous reaction medium leaving extraction zone C through line 12 is recycled to the hydroxyl ammonium reactor A, through lines 13, 14, 15, and 1. A part of the aqueous reaction medium leaving the extraction zone C through line 12 is tapped for absorbing and oxidizing nitrogen oxides. This part of the aqueous reaction medium is fed through line 16 to absorption column D, in which nitrogen oxides are absorbed, which are produced in reactor E by ammonia combustion and fed through line 18 to absorption column D. In column D nitric acid is produced from absorbed nitrogen oxides by a further reaction with water from the aqueous reaction medium. The aqueous reaction medium enriched with nitric acid passes from column D to bleaching column F through line 18. In column F residual nitrogen oxides are oxidized into nitric acid. Accordingly, the nitric acid concentration is increased in the aqueous reaction medium leaving column F through line 19. Optionally an additional amount of nitric acid can be supplied through line 20 and mixed with the acidic aqueous solution comprising nitric acid passing through line 19. The thus obtained second acidic aqueous solution comprising nitric acid passing through line 21 is mixed with the aqueous reaction medium passing through line 14, the first acidic aqueous solution. Optionally an additional amount of nitric acid can be supplied through line 22 and mixed with the third acidic aqueous solution passing through line 15. Subsequently the thus obtained third or fourth acidic aqueous solution is fed to the hydroxyl ammonium reactor through line 1, completing the cycle. The first acidic aqueous solution passing through line 13 may be enriched with hydroxyl ammonium by adding an aqueous solution comprising hydroxylammonium through line 16, which may be tapped from line 5.

Preferably, this process is carried out continuously.

The invention will be elucidated by the following examples, however these are not intended to limit the scope of the invention in any way.

EXAMPLES

In all examples the hydroxyl ammonium concentration in the acidic aqueous solution was determined by a potentiometric titration with $K_3Fe(CN)_6$.

In all examples the total acid concentration in the acidic aqueous solution was determined by titration up to the first equivalence point of $H_3PO_4$ (approx. pH 4.2) with NaOH.

In all examples the phosphate concentration in the acidic aqueous solution was determined by titration with $La(NO_3)_3$.

In all examples decomposition of hydroxyl ammonium was monitored by monitoring gas evolution in the third acidic aqueous solution with a bubbler.

Comparative Experiment A

A glass reactor equipped with baffles and a turbine stirrer was filled with 40 ml of a first acidic aqueous solution, comprising per litre 0.029 mole hydroxyl ammonium, 2.67 mole total acid and 4.51 mole phosphate. This first acidic aqueous solution was heated to 65 °C under a continuous stream of nitrogen gas and vigorous stirring (600 rpm). After the solution has reached the desired temperature the supply of nitrogen was stopped and 30 ml of a second acidic aqueous solution, comprising per litre no hydroxyl ammonium, 7.53 mole total acid and 2.83 mole phosphate, was mixed with the first acidic aqueous solution drop wise. At a certain point during the addition of the second acidic aqueous solution decomposition of hydroxyl ammonium starts, which was observed by a vigorous gas evolution. After the addition of the second acidic aqueous solution was completed and the gas evolution in the third acidic aqueous solution having a temperature of 65 °C had stopped the hydroxyl ammonium concentration in the third acidic aqueous solution was determined by titration and appeared to be < 0.001 mol/l whereas the calculated final hydroxyl ammonium concentration is 0.017 mol/l. In this example decomposition of hydroxyl ammonium occurred. In the third acidic aqueous solution of this comparative Experiment the calculated total acid concentration is 4.75 mol/l and the calculated phosphate concentration is 3.79 mol/l. Thus in the third acidic aqueous solution the total acid concentration minus the phosphate concentration is 0.96 mol/l. The calculated value for

$0.523 * \ln([\text{hydroxyl ammonium}]/1.25) + 422/(T + 81)$ is 0.63. Thus this comparative Experiment demonstrates decomposition of hydroxyl ammonium in the case that the total acid concentration minus the phosphate concentration in the third acidic aqueous solution is higher than $0.523 * \ln([\text{hydroxyl ammonium}]/1.25) + 422/(T + 81)$.

## Example I

Comparative Experiment A was repeated except that instead of 30 ml, 20 ml of the second acidic aqueous solution was mixed with the first acidic aqueous solution. In this case no vigorous gas evolution could be observed. In the third acidic aqueous solution having a temperature of 65 °C the hydroxyl ammonium concentration determined by titration was 0.018 mol/l, which is equal to the calculated value of 0.019. The calculated total acid concentration minus the calculated phosphate concentration in the third acidic aqueous solution of this Example is 0.3 mol/l. The calculated value for $0.523 * \ln([\text{hydroxyl ammonium}]/1.25) + 422/(T + 81)$ is 0.7. Thus this example demonstrates that no decomposition of hydroxyl ammonium occurs in the third acidic aqueous solution when the total acid concentration minus the phosphate concentration in the third acidic aqueous solution is lower than

$$0.523 * \ln([\text{hydroxyl ammonium}]/1.25) + 422/(T + 81).$$

## Example II

Comparative Experiment A was repeated except that the third acidic aqueous solution was heated to 35 °C. No vigorous gas evolution could be observed. In the third acidic aqueous solution the hydroxyl ammonium concentration determined by titration was 0.017 mol/l, which is equal to the calculated hydroxyl ammonium concentration, being 0.017 mol/l. The calculated total acid concentration minus the calculated phosphate concentration in the third acidic aqueous solution of this Example is 1.2 mol/l. The calculated value for $0.523 * \ln([\text{hydroxyl ammonium}]/1.25) + 422/(T + 81)$ is 1.4. Thus this Example demonstrates that no decomposition of hydroxyl ammonium occurs in the third acidic aqueous solution at a temperature of 35 °C when the total acid concentration minus the phosphate concentration in the third acidic aqueous solution is lower than

$$0.523 * \ln([\text{hydroxyl ammonium}]/1.25) + 422/(T + 81).$$

## Example III

Comparative Experiment A was repeated except that as first acidic aqueous solution, was used a solution comprising per litre 0.146 mole hydroxyl ammonium, 2.33 mole total acid and 3.74 mole phosphate. No vigorous gas evolution could be observed. In the third acidic aqueous solution having a temperature of 65 °C the hydroxyl ammonium concentration determined by titration was 0.08 mol/l, which is equal to the calculated hydroxyl ammonium concentration, being 0.08 mol/l. The calculated total acid concentration minus the calculated phosphate concentration in the third acidic aqueous solution of this Example is 1.2 mol/l. The calculated value for $0.523 * \ln([\text{hydroxyl ammonium}]/1.25) + 422/(T + 81)$ is 1.5. Thus this Example demonstrates that no decomposition of hydroxyl ammonium occurs in the third acidic aqueous solution when the total acid concentration minus the phosphate concentration in the third acidic aqueous solution is lower than $0.523 * \ln([\text{hydroxyl ammonium}]/1.25) + 422/(T + 81)$.

Comparative Experiment B

Example I was repeated except that 20 ml of the second acidic aqueous solution was added at once to the first acidic aqueous solution without stirring. In this case a slow gas evolution could be observed, which stopped when after 2 hours stirring was started. In the third acidic aqueous solution the hydroxyl ammonium concentration determined by titration was 0.011 mol/l, the temperature is 65 °C and the calculated hydroxyl ammonium concentration 0.019. The calculated total acid concentration minus the calculated phosphate concentration in the third acidic aqueous solution of this Example is 0.3 mol/l. The calculated value for 0.523 ∗ ln([hydroxyl ammonium]/1.25) + 422/(T + 81) is 0.7. This comparative Example demonstrates that even if the total acid concentration minus the phosphate concentration in the third acidic aqueous solution is lower than 0.523 ∗ ln([hydroxyl ammonium]/1.25) + 422/(T + 81) a slow decomposition of hydroxyl ammonium occurs in the third acidic aqueous solution when no mixing is performed.

Examples IV-XXIV

Comparative Experiment A was repeated except that different first and second acidic aqueous solutions and temperatures were used and that the addition of the second acidic aqueous solution was stopped at the moment gas evolution started. At that moment the amount of added second acidic aqueous solutions was determined, the total acid concentration minus the phosphate concentration in the third acidic aqueous solution was calculated, the value for 0.523 ∗ ln([hydroxyl ammonium]/1.25) + 422/(T + 81 )in the third acidic aqueous solution was calculated and the amount of hydroxyl ammonium was calculated. All data are given in Tables 1-3. These Examples show that decomposition of hydroxyl ammonium starts at the point where the total acid concentration minus the phosphate concentration in the third acidic aqueous solution is equal to

$$0.523 * \ln([\text{hydroxyl ammonium}]/1.25) + 422/(T + 81).$$

**Table 1.** First acidic aqueous solution

| First acidic aqueous solution | Amount of hydroxyl ammonium in mol/l | Total acid concentration in mol/l | Phosphate concentration in mol/l |
|---|---|---|---|
| A1 | 0.090 | 2.39 | 3.80 |
| A2 | 0.029 | 2.67 | 4.51 |
| A3 | 0.146 | 2.33 | 3.74 |
| A4 | 0.020 | 2.74 | 4.55 |

**Table 2.** Second acidic aqueous solution

| Second acidic aqueous solution | Amount of hydroxyl ammonium in mol/l | Total acid concentration in mol/l | Phosphate concentration in mol/l |
|---|---|---|---|
| B1 | 0 | 5.79 | 2.50 |
| B2 | 0 | 7.53 | 2.83 |
| B3 | 0 | 8.81 | 1.90 |
| B4 | 0 | 7.70 | 2.90 |

**Table 3.** Comparison of

the amount of [total acid]–[phosphate] with 0.523*ln([hydroxyl ammonium]/1.25)+422/(T+81)

| Example | First acidic aqueous solution | Amount of first Acidic aqueous solution in ml | Second acidic aqueous solution | Amount of second Acidic aqueous solution in ml | T in °C | [hydroxyl ammonium] in third acidic aqueous solution | Amount of [total acid] – [phosphate] in third acidic aq. solution | 0.523*ln([hydroxyl ammonium]/1.25) + 422/(T+81) |
|---|---|---|---|---|---|---|---|---|
| IV | A1 | 40.0 | B1 | 90.2 | 25 | 0.028 | 1.9 | 2.0 |
| V | A1 | 40.0 | B1 | 75.5 | 35 | 0.031 | 1.7 | 1.7 |
| VI | A1 | 40.0 | B1 | 66.5 | 45 | 0.034 | 1.5 | 1.5 |
| VII | A1 | 40.0 | B1 | 60.0 | 55 | 0.036 | 1.4 | 1.3 |
| VIII | A1 | 40.0 | B3 | 33.6 | 25 | 0.049 | 2.4 | 2.3 |
| IX | A1 | 40.0 | B3 | 29.0 | 35 | 0.052 | 2.1 | 2.0 |
| X | A1 | 40.0 | B3 | 24.5 | 45 | 0.056 | 1.8 | 1.7 |
| XI | A1 | 40.0 | B3 | 22.5 | 55 | 0.057 | 1.6 | 1.5 |
| XII | A2 | 40.0 | B2 | 33.0 | 45 | 0.016 | 1.1 | 1.0 |
| XIII | A2 | 40.0 | B2 | 29.8 | 55 | 0.016 | 1.0 | 0.8 |
| XIV | A2 | 40.0 | B2 | 25.5 | 65 | 0.017 | 0.7 | 0.7 |
| XV | A2 | 40.0 | B2 | 22.2 | 75 | 0.018 | 0.5 | 0.5 |
| XVI | A3 | 40.0 | B3 | 42.0 | 25 | 0.071 | 2.9 | 2.5 |
| XVII | A3 | 40.0 | B3 | 36.8 | 35 | 0.076 | 2.6 | 2.2 |
| XVIII | A3 | 40.0 | B3 | 29.4 | 45 | 0.084 | 2.1 | 1.9 |
| XIX | A3 | 40.0 | B3 | 27.4 | 55 | 0.086 | 2.0 | 1.7 |
| XX | A4 | 40.0 | B4 | 39.4 | 25 | 0.010 | 1.5 | 1.5 |
| XXI | A4 | 40.0 | B4 | 29.2 | 45 | 0.011 | 1.0 | 0.9 |
| XXII | A4 | 40.0 | B4 | 25.0 | 55 | 0.012 | 0.7 | 0.7 |
| XXIII | A4 | 40.0 | B4 | 21.5 | 65 | 0.013 | 0.5 | 0.5 |
| XXIV | A4 | 40.0 | B4 | 19.2 | 75 | 0.013 | 0.3 | 0.3 |

Example XXV

Comparative Experiment A was repeated except that glass reactor equipped with baffles and a turbine stirrer was filled with 75 ml of a first acidic aqueous solution, comprising per litre 1.58 mole hydroxyl ammonium, 0.74 mole total acid and 3.76 mole phosphate. Under vigorous stirring (600 rpm) at a temperature of 60 °C 120 ml of a mixture of 6 parts of a second acidic aqueous solution, comprising per litre no hydroxyl ammonium, 7.70 mole total acid and 2.90 mole phosphate and one part of a 65% aqueous nitric acid solution were mixed with the first acidic aqueous solution. At the moment gas evolution started the calculated hydroxyl ammonium concentration in the third acidic aqueous solution having a temperature of 60 °C was 0.61 mol/l. The calculated total acid concentration minus the calculated phosphate concentration in the third acidic aqueous solution of this Example is 2.7 mol/l. The calculated value for 0.523 ∗ ln([hydroxyl ammonium]/1.25) + 422/(T + 81) is 2.6. This Experiment demonstrates that in the third acidic aqueous solution comprising per litre 0.61 mole hydroxyl ammonium, obtained by mixing a first acidic aqueous solution with a second acidic aqueous solution which is obtained by mixing an acidic aqueous solution comprising nitric acid and phosphate with a 65% nitric acid solution, decomposition of hydroxyl ammonium starts at the moment that the total acid concentration minus the phosphate concentration in the third acidic aqueous solution is equal to 0.523 ∗ ln([hydroxyl ammonium]/1.25) + 422/(T + 81).

Example XXVI

Example XXV was repeated except that for the second acidic aqueous solution a 65% aqueous nitric acid solution is used which was added drop wise to the first acidic aqueous solution until gas evolution started. At the moment gas evolution started 41 ml of the 65% aqueous nitric acid solution has been added and the calculated hydroxyl ammonium concentration in the third acidic aqueous solution having a temperature of 60 °C was 1.02 mol/l. The calculated total acid concentration minus the calculated phosphate concentration in the third acidic aqueous solution of this Example is 3.2 mol/l. The calculated value for 0.523 ∗ ln([hydroxyl ammonium]/1.25) + 422/(T + 81) is 2.9. This Example demonstrates that if the concentration of hydroxyl ammonium in the third acidic aqueous solution is 1.02 mol/l and the second acidic aqueous solution, being a 65% aqueous nitric acid solution, is mixed drop wise with the first acidic aqueous solution no decomposition occurs until the total acid concentration minus the phosphate concentration in the third acidic aqueous solution is equal to 0.523 ∗ ln([hydroxyl ammonium]/1.25) + 422/(T + 81).

Comparative Experiment C

Comparative Experiment A was repeated except that the glass reactor equipped with baffles and a turbine stirrer was filled with 70 ml of a first acidic aqueous solution, comprising per litre 0.020 mole hydroxyl ammonium, 2.74 mole total acid and 4.55 mole phosphate. Under vigorous stirring (600 rpm) at a temperature of 45 °C 30 ml of a second acidic aqueous solution, comprising per litre no hydroxyl ammonium, 7.70 mole total acid and 2.90 mole phosphate and 6 ml of a 65% aqueous nitric acid solution were mixed with the first acidic aqueous solution. Gas evolution was observed. After the gas evolution had stopped the hydroxyl ammonium concentration in the third acidic aqueous solution was determined by titration and appeared to be < 0.001 mol/l. The calculated hydroxyl ammonium concentration in the third acidic aqueous solution having a temperature of 45 °C was 0.013 mol/l. The calculated total acid concentration minus the calculated phosphate concentration in the third acidic aqueous solution of this comparative Experiment is 1.0 mol/l and the calculated value for 0.523 ∗ ln([hydroxyl ammonium]/1.25) + 422/(T + 81) is 1.0. This comparative Experiment demonstrates that in the third acidic aqueous solution having a temperature of 45 °C and a hydroxyl ammonium concentration of 0.013 mol/l, obtained by mixing a first acidic aqueous solution with a second acidic aqueous solution which is obtained by mixing an acidic aqueous solution comprising nitric acid and phosphate with a 65% nitric acid solution, decomposition of hydroxyl ammonium occurs when the total acid concentration minus the phosphate concentration in the third acidic aqueous solution is equal to 0.523 ∗ ln([hydroxyl ammonium]/1.25) + 422/(T + 81).

Example XXVII

Comparative Experiment C was repeated except that the hydroxyl ammonium concentration in the first acidic aqueous solution has been increased by adding 5 ml of an aqueous hydroxyl ammonium solution, comprising per litre 1.58 mole hydroxyl ammonium, 0.74 mole total acid and 3.76 mole phosphate to 70 ml of a first acidic aqueous solution comprising per litre 0.020 mole hydroxyl ammonium, 2.74 mole total acid and 4.55 mole phosphate. No gas evolution was observed. The calculated hydroxyl ammonium concentration in the third acidic aqueous solution having a temperature of 45 °C was 0.084 mol/l. The calculated total acid concentration minus the calculated phosphate concentration in the third acidic aqueous solution of this Example is 0.8 mol/l and the calculated value for 0.523 ∗ ln([hydroxyl ammonium]/1.25) + 422/(T + 81) is 1.9. This Example demonstrates that increasing the amount of hydroxyl ammonium in the third acidic aqueous solution in such a way that the total acid concentration minus the phosphate concentration in the third acidic aqueous solution is lower than 0.523 ∗ ln([hydroxyl ammonium]/1.25) + 422/(T + 81), results in preventing decomposition of hydroxyl ammonium in the third acidic aqueous solution.

**Claims**

1. Process for mixing a first acidic aqueous solution comprising hydroxyl ammonium and phosphate with a second acidic aqueous solution comprising nitric acid at a temperature between 20 and 80 °C resulting in a third acidic aqueous solution comprising hydroxyl ammonium, phosphate and nitric acid wherein in the third acidic aqueous solution the total acid concentration minus the phosphate concentration is lower than $0.523 * \ln([\text{hydroxyl ammonium}]/1.25) + 422/(T + 81)$ whereby [hydroxyl ammonium] is the concentration of hydroxyl ammonium in the third acidic aqueous solution, T is the temperature of the third acidic aqueous solution expressed in °C and all concentrations are expressed in mol/l.

2. Process according to claim 1 wherein the second acidic aqueous solution is obtained by absorbing and oxidizing nitrogen oxides in a part of the first acidic aqueous solution.

3. Process according to claim 1 wherein a part of the second acidic aqueous solution is obtained by absorbing and oxidizing nitrogen oxides in a part of the first acidic aqueous solution.

4. Process according to any one of claims 1-3 wherein the third acidic aqueous solution is mixed with an acidic aqueous solution comprising nitric acid at a temperature between 20 and 80 °C resulting in a fourth acidic aqueous solution comprising hydroxyl ammonium, phosphate and nitric acid whereby the total acid concentration minus the phosphate concentration in the fourth acidic aqueous solution is lower than $0.523 * \ln([\text{hydroxyl ammonium}]/1.25) + 422/(T + 81)$ wherein [hydroxyl ammonium] is the concentration of hydroxyl ammonium in the fourth acidic aqueous solution, T is the temperature of the fourth acidic aqueous solution expressed in °C and all concentrations are expressed in mol/l.

5. Process according to any one of claims 1-4 wherein the third and/or fourth acidic aqueous solution is used as an aqueous reaction medium for the production of hydroxyl ammonium in a hydroxyl ammonium reactor by catalytic reduction of nitrate with hydrogen.

6. Process according to claim 5 wherein the aqueous reaction medium leaving the hydroxyl ammonium reactor is cycled to a cyclohexanone oxime reactor and back to the hydroxyl ammonium reactor whereby in the cyclohexanone oxime reactor cyclohexanone oxime is produced from hydroxyl ammonium and cyclohexanone.

7. Process according to claim 6 wherein the aqueous reaction medium cycled from the cyclohexanone oxime reactor is used as the first acidic aqueous solution.

8. Process according to any one of claims 6-7 wherein the mixing is performed before the third or fourth acid aqueous solution is fed to the hydroxyl ammonium reactor.

9. Process according to any one of claims 1-8 wherein the mixing of the first and second acidic aqueous solution and/or the mixing of the third acidic aqueous solution with an acidic aqueous solution comprising nitric acid is performed in a static mixer.

10. Process according to any one of claims 5-8 wherein the mixing of the first and second acidic aqueous solution and/or the mixing of the third acidic aqueous solution with an acidic aqueous solution comprising nitric acid is performed in the hydroxyl ammonium reactor.

11. Process according to any one of claims 7-10 wherein hydroxyl ammonium is added to the first acidic aqueous solution before the first acidic aqueous solution is mixed with the second acidic aqueous solution.

12. Process according to claim 10 and claim 11 wherein first or third acidic aqueous solution is enriched with hydroxyl ammonium in the hydroxyl ammonium reactor.

13. Process according to claim 2 or 3 and claim 11 wherein hydroxyl ammonium is added to the first acidic aqueous solution after a part of the first acidic aqueous solution is tapped for absorbing and oxidizing nitrogen oxides.

14. Process according to claim 11 or 13 wherein a part of the aqueous reaction medium leaving the hydroxyl ammonium reactor is used to add hydroxyl ammonium to the first or third acidic aqueous solution.

Figure 1

EP 1 428 792 A1

## EUROPEAN SEARCH REPORT

**European Patent Office**

**Application Number**

EP 02 08 0204

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | WO 01 94296 A (BENNEKER ARNO ;OEVERING HENK (NL); DSM NV (NL); PIETERS PAULUS JOH) 13 December 2001 (2001-12-13)<br>* the whole document *<br>--- | 1-14 | C01B21/14<br>C07C249/08 |
| A | WO 01 94298 A (BLAAUW MARC ;OEVERING HENK (NL); DSM NV (NL); SIMONS ANTONIUS JACO) 13 December 2001 (2001-12-13)<br>* page 9, line 27 - page 10, line 31 *<br>--- | 1-14 | |
| A | GB 1 326 405 A (STAMICARBON) 15 August 1973 (1973-08-15)<br>* example 2 *<br>----- | 1-5 | |

**TECHNICAL FIELDS SEARCHED (Int.Cl.7)**

C01B
C07C

The present search report has been drawn up for all claims

| Place of search | Date of completion of the search | Examiner |
|---|---|---|
| MUNICH | 10 April 2003 | Werner, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 1 428 792 A1

## ANNEX TO THE EUROPEAN SEARCH REPORT
## ON EUROPEAN PATENT APPLICATION NO.

EP 02 08 0204

10-04-2003

| Patent document cited in search report | | Publication date | Patent family member(s) | | Publication date |
|---|---|---|---|---|---|
| WO 0194296 | A | 13-12-2001 | AU | 6440701 A | 17-12-2001 |
| | | | AU | 6440801 A | 17-12-2001 |
| | | | WO | 0194296 A1 | 13-12-2001 |
| | | | WO | 0194297 A1 | 13-12-2001 |
| | | | AU | 6440901 A | 17-12-2001 |
| | | | EP | 1299353 A1 | 09-04-2003 |
| | | | WO | 0194298 A1 | 13-12-2001 |
| WO 0194298 | A | 13-12-2001 | AU | 6440701 A | 17-12-2001 |
| | | | AU | 6440901 A | 17-12-2001 |
| | | | EP | 1299353 A1 | 09-04-2003 |
| | | | WO | 0194296 A1 | 13-12-2001 |
| | | | WO | 0194298 A1 | 13-12-2001 |
| GB 1326405 | A | 15-08-1973 | NL | 6914305 A | 23-03-1971 |
| | | | AT | 308705 B | 25-07-1973 |
| | | | BE | 756358 A1 | 18-03-1971 |
| | | | CA | 933725 A1 | 18-09-1973 |
| | | | CH | 565113 A5 | 15-08-1975 |
| | | | CS | 161115 B2 | 04-05-1975 |
| | | | DE | 2046197 A1 | 01-04-1971 |
| | | | ES | 383810 A1 | 01-03-1973 |
| | | | FR | 2062944 A5 | 02-07-1971 |
| | | | HU | 163775 B | 27-10-1973 |
| | | | JP | 50011878 B | 07-05-1975 |
| | | | RO | 55247 A1 | 20-07-1973 |
| | | | SE | 360339 B | 24-09-1973 |
| | | | ZA | 7006278 A | 27-05-1971 |